# EUROPEAN PATENT SPECIFICATION

(11) **EP 1 492 540 B1**
(45) Date of publication and mention of the grant of the patent: **17.10.2007**
(21) Application number: 03725318.4
(22) Date of filing: 09.04.2003
(51) Int. Cl.: A61K 31/5513, A61K 31/485, A61P 25/04, A61P 43/00

(54) **THE USE OF DEVAZEPIDE AS ANALGESIC AGENT**
VERWENDUNG VON DEVAZEPIDE ALS SCHMERZMITTEL
UTILISATION DU DEVAZEPIDE COMME ANALGESIQUE

(30) Priority: 09.04.2002 GB 0208129
(43) Date of publication of application: 05.01.2005
(73) Proprietor: Panos Therapeutics Limited, Oxford OX44 7PU (GB)
(72) Inventor: JACKSON, Karen, Sheffield S35 2ND (GB)
(74) Representative: Harrison Goddard Foote
(86) International application number: PCT/GB2003/001514
(87) International publication number: WO 2003/086409

(56) References cited:
- EP-A- 0 434 364
- WO-A-99/18967
- DOURISH C T ET AL: "The cholecystokinin receptor antagonist devazepide enhances morphine-induced analgesia but not morphine-induced respiratory depression in the squirrel monkey." THE JOURNAL OF PHARMACOLOGY AND EXPERIMENTAL THERAPEUTICS. UNITED STATES DEC 1990, vol. 255, no. 3, December 1990 (1990-12), pages 1158-1165, XP009013481 ISSN: 0022-3565

## Description

This invention relates to a novel use of a medicament.

International Patent Application No. WO 99/18967 describes pharmaceutical compositions for treating chronic and neuropathic pain which comprises an analgesic amount of an opioid and an opioid potentiating amount of a CCK antagonist. WO '967 describes the use of both CCK-A (CCK-1) antagonists and CCK-B (CCK-2) antagonists, although it is described that, generally, CCK-B (CCK-2) antagonists are preferred. Moreover, page 2, lines 6 to 8 of WO '967 describes that CCK-A (CCK-1) - antagonists may be suitable, but only at relatively higher dosages.

One specific CCK-A (CCK-1) antagonist which is mentioned is devazepide, which is 3s-(-)1,3-dihydro-3-(2-indolecarbonylamino)-1-methyl-5-phenyl-2H-1,4-benzodiazepin-2-one.

Xu, in "Pain 1994; 56:271-277", describes the effect of pharmacological intervention on a specific type of pain called allodynia, modelled in the rat. Allodynia refers to pain from stimuli which are not normally painful and pain which occurs other than in the area stimulated. It is not synonymous with referred pain. Allodynia is a (clinical) condition in which a normally painless stimulus like touch, warmth, or coolness is perceived as painful.

Xu reported a specific pain response, which was presumed to be allodynic, that responded to a CCK-B (CCK-2) antagonist in the absence of opioids. This was thought to be mediated by endogenous opioids, particularly since the response was reversed by the opioid receptor antagonist, naloxone. However, importantly, the pain response observed by Xu did not respond to a CCK-A (CCK-1) antagonist.

International Patent Application No. WO 99/18967 describes a pharmaceutical formulation comprising a CCK antagonist, such as devazepide (Devacade®), an opioid and a biphasic carrier, comprising a glyceride derivative organic phase. The use of the CCK antagonist is intended to block the CCK receptors thereby reversing or preventing the development of opioid tolerance in patients and potentiating the analgesic effect of the opioid.

However, in clinical studies we have surprisingly found that, in some patients, opioid therapy was able to be removed completely. These patients experienced pain relief on administration of devazepide only. Thus it is a novel aspect of the present invention to be able to use devazepide as an analgesic therapy in its own right, rather than solely as an adjunct to opioid therapy.

Thus, according to the invention we provide the use of devazepide in the manufacture of a medicament suitable for the treatment of a patient requiring analgesic therapy which comprises the administration of a therapeutically effective amount of an opioid analgesic and the separate, simultaneous or sequential administration of an analgesically effective amount of devazepide characterised in that the treatment comprises;
(i) initial analgesic dosages of an opioid and devazepide; and
(ii) subsequent dosages of devazepide only.

In this aspect of the invention a variety of opioids may be used. Thus, the opioid may be selected from those which are effective analgesics and particularly those which need to be administered at relatively high or increasing doses. Examples include morphine, or a salt thereof such as the sulphate, chloride or hydrochloride, or the other 1,4-hydroxymorphinan opioid analgesics such as meperidine, butorphanol or pentazocine, or morphine-6-glucuronide, codeine, dihydrocodeine, diamorphine, dextropropoxyphene, pethidine, fentanyl, alfentanil, alphaprodine, buprenorphine, dextromoramide, diphenoxylate, dipipanone, heroin (diacetylmorphine), hydrocodone (dihydrocodeinone), hydromorphone (dihydromorphinone), levorphanol, meptazinol, methadone, metopon (methyldihydromorphinone), nalbuphine, oxycodone (dihydrohydroxycodeinone), oxymorphone (dihydrohydroxymorphinone), phenadoxone, phenazocine, remifentanil, tramadol, or a salt of any of these. Naloxone is also included within the definition of an opioid. Especially preferred analgesics which may be mentioned are hydromorphone, oxycodone, morphine, e.g. morphine sulphate and fentanyl. In a preferred embodiment of the invention the analgesic is morphine or morphine sulphate. In a further preferred embodiment the opioid is fentanyl or a salt thereof.

In the use of the invention the devazepide and/or the opioid may be administered using any methods conventionally known *per se.* Thus, such methods would include, but shall not be limited to, administration intravenously, intra-arterially, orally, intrathecally, intranasally, intrarectally, intramuscularly/subcutaneously, by inhalation and by transdermal patch. When the devazepide and/or opioid is administered intravenously, it may, for example, be as an intravenous bolus or a continuous intravenous infusion. When the devazepide and/or the opioid is administered subcutaneously, it may for example be by subcutaneous infusion. Preferably, the opioid and/or devazepide are administered intravenously or orally. Oral administration is especially preferred. In a further preferred embodiment the opioid may be administered by a transdermal patch. When a transdermal patch is used, the preferred opioid is fentanyl or a salt thereof.

Preferentially, the opioid and the devazepide will be administered using the same mode of administration. Thus, for example, when the opioid is administered intravenously then the devazepide will be administered intravenously also. Similarly, when the opioid is administered orally then the devazepide will be administered orally also. However, it is within the scope of the invention for either the opioid to be administered orally and the devazepide to be administered intravenously or vice versa.

In this use a patient may be started on an analgesic therapy which comprises administering an effective amount of an opioid in conjunction with devazepide. As the treatment becomes effective the opioid dose may be diminished, eventually to zero, e.g. wherein devazepide is the sole therapy.

In the use of the invention it should be understood that the devazepide will have an analgesic therapeutic effect. We especially provide the use as hereinbefore described wherein the medicament is effective in the treatment or alleviation of neuropathic pain.

Thus, in the use of the invention the daily dosage of devazepide may vary depending upon, *inter alia,* the weight of the patient, the method of administration, etc. In patients that are suffering serious disorders, such as cancer patients, the weight of the patient may be very low and therefore the dosage of devazepide consequentially may be low. Thus the daily dosage of devazepide may be up to 0.7 mg/kg/day. Preferably, the daily dosage of devazepide may be from 25 µg/kg/day to 0.7 mg/kg/day, more preferably from 50 µg/kg/day to 0.5 mg/kg/day. For oral administration the daily dosage of devazepide may be from 0.07 mg/kg/day to 0.7 mg/kg/day, preferably 0.07 mg/kg/day to 0.29 mg/kg/day. For intravenous administration the dosage of devazepide is preferably 50 µg/kg/day to 0.5 mg/kg/day

In the use of the invention the dosage of the opioid analgesic administered may vary depending upon, inter alia, the nature of the opioid analgesic, the weight of the patient, the method of administration, etc. Thus, for example, the dosage of, e.g. an opioid, such as morphine, may be from 5 to 2000mg daily. A particular dosage which may be mentioned is from 10 to 240mg daily. A daily dosage of morphine may be from 5 to 100mg or occasionally up to 500mg.

When the composition used in the method of the invention includes a filler, the composition may generally comprise devazepide and a surfactant, in the ratio as hereinbefore described, with the remainder of the composition being made up with a filler.

A preferred embodiment of the invention comprises use wherein a composition as hereinbefore described is filled into a capsule. Any conventionally known materials may be used for the capsule, however a preferred material is gelatin.

Thus, for example, in one embodiment of the invention the composition may be made up into a capsule formulation, e.g. with a fill weight of 150 mg ± 5% by weight or 300 mg ± 5% by weight. In the one preferred embodiment, the capsule formulation may comprise 1.25mg devazepide, and 148.75 mg of a filler or other excipients, e.g. corn starch. In a further preferred embodiment, the capsule formulation may comprise 2.5mg devazepide, and 297.5 mg of a filler or other excipients, e.g. corn starch.

Thus, such fillers may be selected from the group lactose, mannitol, talc, magnesium stearate, sodium chloride, potassium chloride, citric acid, spray-dried lactose, hydrolysed starches, directly compressible starch, microcrystalline cellulose, cellulosics, sorbitol, sucrose, sucrose-based materials, icodextrin, calcium sulphate, dibasic calcium phosphate and dextrose. A preferred filler is starch, e.g. corn starch.

When the composition of the invention includes a filler, the size of the devazepide and filler particles may be the same or different. However, in a preferred embodiment the sizes of the devazepide and filler particles will differ. Preferentially, the devazepide and/or the filler may be of reduced particle size, e.g. by milling.

The devazepide used in the invention is the S enantiomer, preferentially, the S enantiomer wherein the level of R enantiomer, which may be present as an impurity, is not greater than 1.5% w/w.

The invention will now be illustrated by way of example only and with reference to the accompanying drawings in which;
Figure 1 is a graph comparing respective dosages of devazepide and opioid (morphine) over a given period of time; and
Figure 2 is a graph comparing respective dosages of devazepide and opioid (dihydrocodeine) over a given period.

### Example 1

### Clinical assessment study

A research programme has included a double blind, double dummy, randomised, crossover study of a single dose of either 1.25mg devazepide, 5.0 mg devazepide or placebo. Patients who took part in the study had pain with a neuropathic element, and were taking regular, stable doses of strong opioids. Following completion of the study those patients who, in the opinion of their Clinical Investigator, had gained benefit from participation were given the opportunity to consent to continue receiving devazepide treatment for a period of up to six months.

### Study design

This continuation study was a multicentre, open label study of devazepide at twice daily doses of 1.25mg, 2.5mg and 5.0 mg.

### Study Objective

The primary objective of this study was to compare descriptive and visual analogue scale (VAS) assessments of pain and pain relief in patients with neuropathic pain.

### Methods

At the end of the previous randomised trial, patients received 1.25mg devazepide twice daily for an initial period of one week. After this initial one week period, the dose of devazepide was reviewed and increased, if necessary, to 2.5 mg twice daily and thereafter to 5.0 mg twice daily as required. Devazepide treatment was continued for a period of up to six months.

During the study patients were required to remain on stable, regular doses of opioids at a dose prescribed by the investigator.

### Study assessments

Patients were assessed at clinic visits at week 1, week 2 (dose escalation) and thereafter at routine monthly clinic visits.

At weekly intervals for the first eight weeks and at monthly intervals thereafter, patients recorded pain and global pain relief using VAS and descriptive pain questionnaires. The questionnaires were returned to the Investigator at the monthly visits.

At each clinic visit the Investigator assessed safety and the patients' pain relief, reviewed the dosage, and decided if devazepide treatment should be continued.

### Results

Seventeen patients elected to stay on devazepide by entering the continuation study and received devazepide at 1.25mg, 2.5mg or 5.0 mg twice daily for up to 26 weeks.

Of these patients, ten appeared to achieve long-term pain relief (5 - 26 weeks) with devazepide. Despite the requirement to remain on stable, regular doses of opioids at the dose prescribed by the investigator, several patients reduced markedly or reduced to zero their daily opioid dose.

The graph of Figure 1 illustrates the reduction in opioid (morphine) dosage which may be achieved by administration of devazepide over a period of five months. The patient(s) commenced on 50 mg morphine per day.

Figure 2 illustrates the trend with a weaker opioid, dihydrocodeine. The patient(s) commenced on 120 mg dihydrocodeine per day.

## Claims

1. The use of devazepide in the manufacture of a medicament for the treatment of a patient by analgesic therapy which comprises the administration of a therapeutically effective amount of an opioid analgesic and the separate, simultaneous or sequential administration of an analgesically effective amount of devazepide **characterised in that** the treatment comprises;
(i) initial analgesic dosages of an opioid and devazepide; and
(ii) subsequent dosages of devazepide only.

2. The use according to claim 1 **characterised in that** the opioid is selected from the group morphine, or a salt thereof such as the sulphate, chloride or hydrochloride, or the other 1,4-hydroxymorphinan opioid analgesics such as meperidine, butorphanol or pentazocine, or morphine-6-glucuronide, codeine, dihydrocodeine, diamorphine, dextropropoxyphene, pethidine, fentanyl, alfentanil, alphaprodine, buprenorphine, dextromoramide, diphenoxylate, dipipanone, heroin (diacetylmorphine), hydrocodone (dihydrocodeinone), hydromorphone (dihydromorphinone), levorphanol, meptazinol, methadone, metopon (methyldihydromorphinone), nalbuphine, oxycodone (dihydrohydroxycodeinone), oxymorphone (dihydrohydroxymorphinone), phenadoxone, phenazocine, remifentanil, tramadol, or a salt of any of these, or a combination of the aforementioned compounds.

3. The use according to claim 1 **characterised in that** the opioid is naloxone.

4. The use according to claim 2 **characterised in that** the analgesic is selected from the group hydromorphone, oxycodone, morphine and fentanyl, or a salt of any of these.

5. The use according to claim 4 **characterised in that** the opioid is fentanyl or a salt thereof.

6. The use according to claim 4 **characterised in that** the opioid is selected from the group morphine and morphine sulphate.

7. The use according to claim 1 **characterised in that** some pain episodes may be treated by administering an additional dosage or dosages of an opioid.

8. The use according to claim 1 **characterised in that** the method of delivery of the devazepide and/or the opioid is selected from the group, administration intravenously, intra-arterially, orally, intrathecally, intranasally, intrarectally, intramuscularly/subcutaneously, by inhalation and by transdermal patch.

9. The use according to claim 8 **characterised in that** the opioid and the devazepide are administered using the same mode of administration.

10. The use according to claim 8 **characterised in that** the devazepide and/or the opioid is administered intravenously.

11. The use according to claim 10 **characterised in that** the intravenous administration is by intravenous bolus or a continuous intravenous infusion.

12. The use according to claim 8 **characterised in that** the devazepide and/or the opioid is administered subcutaneously.

13. The use according to claim 12 **characterised in that** the subcutaneous administration is as a subcutaneous infusion.

14. The use according to claim 8 **characterised in that** the devazepide and/or the opioid is administered orally.

15. The use according to claim 1 **characterised in that** the devazepide is administered orally.

16. The use according to claim 10 **characterised in that** the opioid is administere intravenously and the devazepide is administered intravenously.

17. The use according to claim 14 **characterised in that** the opioid is administered orally and the devazepide is administered orally.

18. The use according to claim 8 **characterised in that** the opioid is administered by intravenous administration or oral administration.

19. The use according to claim 15 **characterised in that** the composition comprises devazepide and a surfactant with the remainder of the composition being made up with a filler.

20. The use according to claims 1 or 19 **characterised in that** the composition is filled into a capsule.

21. The use according to claim 20 **characterised in that** the capsule is a gelatin capsule.

22. The use according to claim 8 **characterised in that** the opioid is administered by transdermal patch.

23. The use according to claim 22 **characterised in that** the opioid is fentanyl, or a salt thereof.

24. The use according to claim 1 **characterised in that** the daily dosage of devazepide is up to 0.7 mg/kg/day.

25. The use according to claim 24 **characterised in that** the daily dosage of devazepide is from 25 *µ*g/kg/day to 0.7 mg/kg/day.

26. The use according to claim 24 **characterised in that** the daily dosage of devazepide is from 50 µg/kg/day to 0.5 mg/kg/day.

27. The use according to claim 24 **characterised in that** the dosage of devazepide is an oral dosage.

28. The use according to claim 27 **characterised in that** for oral administration the daily dosage of devazepide is from 0,07 mg/kg/day to 0.7 mg/kg/day.

29. The use according to claim 27 **characterised in that** the devazepide is administered orally and the daily dosage of devazepide is from 0.07 mg/kg/day to 0.29 mg/kg/day.

30. The use according to claim 10 **characterised in that** the devazepide is administered intravenously and the daily dosage of devazepide is from 50 µg/kg/day to 0.5 mg/kg/day.

31. The use according to claim 1 **characterised in that** the daily dosage of the opioid is from 5 to 2000mg daily.

32. The use according to claim 31 **characterised in that** the dosage of the opioid is from 10 to 240mg daily.

33. The use according to claim 31 **characterised in that** the daily dosage of the opioid is from 5 to 100mg daily.

34. The use according to claim 1 **characterised in that** the devazepide used in the method of the invention is substantially the S enantiomer.

35. The use according to claim 34 **characterised in that** the level of R enantiomer, which may be present as an impurity, is not greater than 1,5% w/w.

## Patentansprüche

1. Verwendung von Devazepid bei der Herstellung eines Arzneimittels für die Behandlung eines Patienten durch schmerzstillende Therapie, welche die Verabreichung einer therapeutisch wirksamen Menge eines Opioid-Schmerzmittels und die getrennte, gleichzeitige oder aufeinander folgende Verabreichung einer analgetisch wirksamen Menge von Devazepid umfasst, **dadurch gekennzeichnet, dass** die Behandlung umfasst:
(i) anfängliche schmerzstillende Dosierungen eines Opioids und von Devazepid; und
(ii) nachfolgende Dosierungen nur von Devazepid.

2. Verwendung nach Anspruch 1, **dadurch gekennzeichnet, dass** das Opioid gewählt ist aus der Gruppe Morphin oder ein Salz davon wie beispielsweise das Sulfat, Chlorid oder Hydrochlorid oder den anderen 1,4-Hydroxymorphinan-Opioid-Schmerzmitteln wie beispielsweise Meperidin, Butorphanol oder Pentazocin, oder Morphin-6-glucuronid, Codein, Dihydrocodein, Diamorphin, Dextropropoxyphen, Pethidin, Fentanyl, Alfentanil, Alphaprodin, Buprenorphin, Dextromoramid, Diphenoxylat, Dipipanon, Heroin (Diactylmorphin), Hydrocodon (Dihydrocodeinon), Hydromorphon (Dihydromorphinon), Levorphanol, Meptazinol, Methadon, Metopon (Methyldihydromorphinon), Nalbuphin, Oxycodon (Dihydrohydroxycodeinon), Oxymorphon (Dihydrohydroxymorphinon), Phenadoxon, Phenazocin, Remifentanil, Tramadol oder ein Salz von einer dieser Verbindungen oder eine Kombination der vorgenannten Verbindungen.

3. Verwendung nach Anspruch 1, **dadurch gekennzeichnet, dass** das Opioid Naloxon ist.

4. Verwendung nach Anspruch 2, **dadurch gekennzeichnet, dass** das Analgetikum (Schmerzmittel) gewählt ist aus der Gruppe Hydromorphon, Oxycodon, Morphin und Fentanyl oder einem Salz irgendeiner dieser Substanzen.

5. Verwendung nach Anspruch 4, **dadurch gekennzeichnet, dass** das Opioid Fentanyl oder ein Salz davon ist.

6. Verwendung nach Anspruch 4, **dadurch gekennzeichnet, dass** das Opioid gewählt ist aus der Gruppe Morphin und Morphinsulfat.

7. Verwendung nach Anspruch 1, **dadurch gekennzeichnet, dass** einige Schmerz-Episoden behandelt werden können durch Verabreichen einer zusätzlichen Dosierung oder zusätzlicher Dosierungen eines Opioids.

8. Verwendung nach Anspruch 1, **dadurch gekennzeichnet, dass** das Verfahren zur Verabreichung des Devazepids und/oder des Opioids gewählt ist aus der Gruppe intravenöse, intra-arterielle, orale, intrathekale, intranasale, intrarektale, intramuskuläre/subkutane Verabreichung, durch Inhalation oder durch ein Transdermal-Pflaster.

9. Verwendung nach Anspruch 8, **dadurch gekennzeichnet, dass** das Opioid und das Devazepid unter Anwendung derselben Verabreichungs-Art verabreicht werden.

10. Verwendung nach Anspruch 8, **dadurch gekennzeichnet, dass** das Devazepid und/oder das Opioid intravenös verabreicht wird/werden.

11. Verwendung nach Anspruch 10, **dadurch gekennzeichnet, dass** die intravenöse Verabreichung eine Verabreichung durch einen intravenösen Bolus oder durch eine kontinuierliche intravenöse Infusion ist.

12. Verwendung nach Anspruch 8, **dadurch gekennzeichnet, dass** das Devazepid und/oder Opioid subkutan verabreicht wird/werden.

13. Verwendung nach Anspruch 12, **dadurch gekennzeichnet, dass** die subkutane Verabreichung eine Verabreichung als subkutane Infusion ist.

14. Verwendung nach Anspruch 8, **dadurch gekennzeichnet, dass** das Devazepid und/oder das Opioid oral verabreicht wird/werden.

15. Verwendung nach Anspruch 1, **dadurch gekennzeichnet, dass** das Devazepid oral verabreicht wird.

16. Verwendung nach Anspruch 10, **dadurch gekennzeichnet, dass** das Opioid intravenös verabreicht wird und das Devazepid intravenös verabreicht wird.

17. Verwendung nach Anspruch 14, **dadurch gekennzeichnet, dass** das Opioid oral verabreicht wird und das Devazepid oral verabreicht wird.

18. Verwendung nach Anspruch 8, **dadurch gekennzeichnet, dass** das Opioid durch intravenöse Verabreichung oder orale Verabreichung verabreicht wird.

19. Verwendung nach Anspruch 15, **dadurch gekennzeichnet, dass** die Zubereitung Devazepid und ein Tensid umfasst, wobei der Rest der Zusammensetzung aus einem Füllstoff besteht.

20. Verwendung nach Anspruch 1 oder 19, **dadurch gekennzeichnet, dass** die Zusammensetzung in eine Kapsel gefüllt wird.

21. Verwendung nach Anspruch 20, **dadurch gekennzeichnet, dass** die Kapsel eine Gelatine-Kapsel ist.

22. Verwendung nach Anspruch 8, **dadurch gekennzeichnet, dass** das Opioid durch ein Transdermal-Pflaster verabreicht wird.

23. Verwendung nach Anspruch 22, **dadurch gekennzeichnet, dass** das Opioid Fentanyl oder ein Salz davon ist.

24. Verwendung nach Anspruch 1, **dadurch gekennzeichnet, dass** die tägliche Dosierung von Devazepid bis zu 0,7 mg/kg/Tag beträgt.

25. Verwendung nach Anspruch 24, **dadurch gekennzeichnet, dass** die tägliche Dosierung von Devazepid von 25 µg/kg/Tag bis 0,7 mg/kg/Tag beträgt.

26. Verwendung nach Anspruch 24, **dadurch gekennzeichnet, dass** die tägliche Dosierung von Devazepid von 50 µg/kg/Tag bis 0,5 mg/kg/Tag beträgt.

27. Verwendung nach Anspruch 24, **dadurch gekennzeichnet, dass** die Dosierung von Devazepid eine orale Dosierung ist.

28. Verwendung nach Anspruch 27, **dadurch gekennzeichnet, dass** für eine orale Verabreichung die tägliche Dosierung an Devazepid von 0,07 mg/kg/Tag bis 0,7 mg/kg/Tag beträgt.

29. Verwendung nach Anspruch 27, **dadurch gekennzeichnet, dass** das Devazepid oral verabreicht wird und die tägliche Dosierung von Devazepid von 0,07 mg/kg/Tag bis 0,29 mg/kg/Tag beträgt.

30. Verwendung nach Anspruch 10, **dadurch gekennzeichnet, dass** das Devazepid intravenös verabreicht wird und die tägliche Dosierung von Devazepid 50 µg/kg/Tag bis 0,5 mg/kg/Tag beträgt.

31. Verwendung nach Anspruch 1, **dadurch gekennzeichnet, dass** die tägliche Dosierung des Opioids 5 bis 2.000 mg täglich beträgt.

32. Verwendung nach Anspruch 31, **dadurch gekennzeichnet, dass** die Dosierung des Opioids 10 bis 240 mg täglich beträgt.

33. Verwendung nach Anspruch 31, **dadurch gekennzeichnet, dass** die tägliche Dosierung des Opioids 5 bis 100 mg täglich beträgt.

34. Verwendung nach Anspruch 1, **dadurch gekennzeichnet, dass** das Devazepid, das in dem Verfahren gemäß der Erfindung verwendet wird, im Wesentlichen das S-Enantiomer ist.

35. Verwendung nach Anspruch 34, **dadurch gekennzeichnet, dass** die Konzentrationsmenge an R-Enantiomer, die als Verunreinigung zugegen sein kann, nicht größer ist als 1,5 % (w/w).

## Revendications

1. Utilisation de dévazépide dans la fabrication d'un médicament destiné au traitement d'un patient par thérapie analgésique, qui comprend l'administration d'une quantité à effet thérapeutique d'un analgésique opioïde et l'administration séparée, simultanée ou séquentielle d'une quantité à effet analgésique de dévazépide, **caractérisée en ce que** le traitement comprend .
(i) l'administration de doses analgésiques initiales d'un opioïde et de dévazépide ; et
(ii) l'administration de doses subséquentes de dévazépide seulement.

2. Utilisation selon la revendication 1, **caractérisée en ce que** l'opioïde est choisi dans le groupe formé par la morphine ou un de ses sels, tel que le sulfate, le chlorure ou le chlorhydrate, ou les autres analgésiques opioïdes du type 1,4-hydroxymorphinane tels que la mépéridine, le butorphanol ou la pentazocine, ou le morphine-6-glucuronide, la codéine, la dihydrocodéine, la diamorphine, le dextropropoxyphène, la péthidine, le fentanyl, l'alfentanil, l'alphaprodine, la buprénorphine, le dextromoramide, le diphénoxylate, la dipipanone, l'héroïne (diacétylmorphine), l'hydrocodone (dihydro-codéinone), l'hydromorphone (dihydromorphinone), le lévorphanol, le meptazinol, la méthadone, le métopon (méthyldihydromorphinone), la nalbuphine, l'oxycodone (dihydrohydroxycodéinone), l'oxymorphone (dihydrohydroxymorphinone), la phénadoxone, la phénazocine, le rémifentanil, le tramadol, ou un sel de n'importe lequel d'entre eux, ou une association des composés susmentionnés.

3. Utilisation selon la revendication 1, **caractérisée en ce que** l'opioïde est la naloxone.

4. Utilisation selon la revendication 2, **caractérisée en ce que** l'analgésique est choisi dans le groupe formé par l'hydromorphone, l'oxycodone, la morphine et le fentanyl, ou un sel de n'importe lequel d'entre eux.

5. Utilisation selon la revendication 4, **caractérisée en ce que** l'opioïde est le fentanyl ou un sel de celui-ci.

6. Utilisation selon la revendication 4, **caractérisée en ce que** l'opioïde est choisi dans le groupe formé par la morphine et le sulfate de morphine.

7. Utilisation selon la revendication 1, **caractérisée en ce que** certaines crises douloureuses peuvent être traitées par l'administration d'une ou plusieurs doses supplémentaires d'un opioïde.

8. Utilisation selon la revendication 1, **caractérisée en ce que** la méthode d'administration du dévazépide et/ou de l'opioïde est choisie dans le groupe formé par l'administration intraveineuse, intra-artérielle, orale, intrathécale, intranasale, intrarectale, intramusculaire/sous-cutanée, par inhalation et par timbre transdermique.

9. Utilisation selon la revendication 8, **caractérisée en ce que** l'opioïde et le dévazépide sont administrés en utilisant le même mode d'administration.

10. Utilisation selon la revendication 8, **caractérisée en ce que** le dévazépide et/ou l'opioïde sont administrés par voie intraveineuse.

11. Utilisation selon la revendication 10, **caractérisée en ce que** l'administration intraveineuse se fait par un bol intraveineux ou par perfusion intraveineuse continue.

12. Utilisation selon la revendication 8, **caractérisée en ce que** le dévazépide et/ou l'opioïde sont administrés par voie sous-cutanée.

13. Utilisation selon la revendication 12, **caractérisée en ce que** l'administration sous-cutanée se fait par perfusion sous-cutanée.

14. Utilisation selon la revendication 8, **caractérisée en ce que** le dévazépide et/ou l'opioïde sont administrés par voie orale.

15. Utilisation selon la revendication 1, **caractérisée en ce que** le dévazépide est administré par voie orale.

16. Utilisation selon la revendication 10, **caractérisée en ce que** l'opioïde est administré par voie intraveineuse et le dévazépide est administré par voie intraveineuse.

17. Utilisation selon la revendication 14, **caractérisée en ce que** l'opioïde est administré par voie orale et le dévazépide est administré par voie orale.

18. Utilisation selon la revendication 8, **caractérisée en ce que** l'opioïde est administré par administration intraveineuse ou par administration orale.

19. Utilisation selon la revendication 15, **caractérisée en ce que** la composition comprend du dévazépide et un agent tensioactif, le reste de la composition étant constitué d'une charge.

20. Utilisation selon la revendication 1 ou 19, **caractérisée en ce que** la composition est introduite dans une capsule.

21. Utilisation selon la revendication 20, **caractérisée en ce que** la capsule est une capsule de gélatine.

22. Utilisation selon la revendication 8, **caractérisée en ce que** l'opioïde est administré par un timbre transdermique.

23. Utilisation selon la revendication 22, **caractérisée en ce que** l'opioïde est le fentanyl ou un sel de celui-ci.

24. Utilisation selon la revendication 1, **caractérisée en ce que** la dose quotidienne de dévazépide est d'au plus 0,7 mg/kg/jour.

25. Utilisation selon la revendication 24, **caractérisée en ce que** la dose quotidienne de dévazépide est de 25 µg/kg/jour à 0,7 mg/kg/jour.

26. Utilisation selon la revendication 24, **caractérisée en ce que** la dose quotidienne de dévazépide est de 50 µg/kg/jour à 0,5 mg/kg/jour.

27. Utilisation selon la revendication 24, **caractérisée en ce que** la dose de dévazépide est une dose orale.

28. Utilisation selon la revendication 27, **caractérisée en ce que**, pour l'administration orale, la dose quotidienne de dévazépide est de 0,07 mg/kg/jour à 0,7 mg/kg/jour.

29. Utilisation selon la revendication 27, **caractérisée en ce que** le dévazépide est administré par voie orale et la dose quotidienne de dévazépide est de 0,07 mg/kg/jour à 0,29 mg/kg/jour.

30. Utilisation selon la revendication 10, **caractérisée en ce que** le dévazépide est administré par voie intraveineuse et la dose quotidienne de dévazépide est de 50 µg/kg/jour à 0,5 mg/kg/jour.

31. Utilisation selon la revendication 1, **caractérisée en ce que** la dose quotidienne de l'opioïde est de 5 à 2000 mg par jour.

32. Utilisation selon la revendication 31, **caractérisée en ce que** la dose de l'opioïde est de 10 à 240 mg par jour.

33. Utilisation selon la revendication 31, **caractérisée en ce que** la dose quotidienne de l'opioïde est de 5 à 100 mg par jour.

34. Utilisation selon la revendication 1, **caractérisée en ce que** le dévazépide utilisé dans la méthode de l'invention est substantiellement l'énantiomère S.

35. Utilisation selon la revendication 34, **caractérisée en ce que** le taux d'énantiomère R, qui peut être présent comme impureté, n'est pas supérieur à 1,5 % en poids/poids.
